# EUROPEAN PATENT APPLICATION

(11) **EP 3 760 860 A1**
(43) Date of publication of application: **06.01.2021**
(21) Application number: 19184596.5
(22) Date of filing: 05.07.2019
(51) Int. Cl.: F03D 9/19, F03D 13/20, C12M 1/107

(54) **ENERGY GENERATION AND ENERGY STORAGE IN A WIND TURBINE STRUCTURE**

(71) Applicant: Siemens Gamesa Renewable Energy A/S, 7330 Brande (DK)
(72) Inventor: Soerensen, Johnny, 6920 Videbæk (DK); Lauritsen, Per Hessellund, 6971 Spjald (DK)
(74) Representative: Aspacher, Karl-Georg

(57) **Abstract**

A wind turbine (1) includes a hollow portion (20) for storing a gas (G), wherein the hollow portion (20) is comprised in a foundation structure (10) or in a tower (2) of the wind turbine (1).

## Description

### Field of invention

The present invention relates to energy generation and energy storage in wind turbines from a source other than the wind.

### Art Background

In some wind turbines applications, it may be necessary or desirable to power the wind turbine in off-grid situations and/or supply electricity to the grid independently from the electricity produced by exploiting the wind. In yet another aspect, it may be necessary or desirable to provide a wind turbine with a source of energy, for example by storing a gas, to be used to generate heat for heating the components of the wind turbine (nacelle and/or tower). Additionally, in off grid extreme situations wind turbines are required to be able to yaw for six hours in order to survive wind gusts when a low pressure storm is passing. An additional source of power may be desirable for powering the yaw system as an UPS (Uninterruptible Power Supply). In offshore wind turbines, it may be further necessary or desirable to provide a fuel for powering offshore vessels, e.g. those used for installation and servicing offshore wind turbines.

It is therefore an object of the present invention to provide a wind turbine with an apparatus and method for storing and/or generating energy for the above described or for other uses.

### Summary of the Invention

The above defined scope is met by the subject matter according to the independent claims. Advantageous embodiments of the present invention are described by the dependent claims.

According to the present invention a wind turbine is provided, which includes a hollow portion for storing a gas, wherein the hollow portion is comprises in a foundation structure or in a tower of the wind turbine.

According to embodiments of the present invention, the gas inside the hollow portion is a biogas (also referred to as "blue-fuel") produced inside the hollow portion used as a bioreactor. A biomass is inserted in the bioreactor and a biogas is generated in the bioreactor and afterwards stored in the bioreactor. The biomass may be a sea based organic material, such as seaweed. According to other embodiments of the present invention, the gas inside the hollow portion is hydrogen produced by an electrolytic hydrogen producing device, which transforms a mass of water, for example sea water, into hydrogen using an electricity source as energy input. The hydrogen producing device may include a cathode and anode system using electricity preferably supplied from the wind turbine. This electricity is by example supplied in a situation with excess power production and/or under negative electricity price periods. According to further embodiments of the present invention, the gas is provided to the hollow portion from an external source, for example through a duct or a pipeline.

The gas stored in the hollow portion may be utilized in a gas generator, powered by the gas to generate electricity. Such gas generator may be used to power the wind turbine in off-grid situations and/or to supply electricity to the grid. The gas may be also used in a gas heater of the wind turbine to generate heat for heating the nacelle and/or tower of the wind turbine. Such heating device is particularly advantageous if the turbine is off-grid and without power. This enables the gas heater to be much smaller than the traditional diesel heaters used in off-grid situations, so that it may be easily integrated in the internal design of the support structure of the wind turbine, e.g. in the foundation, or in the transition piece (TP) or in the tower. Both the gas generator and the gas heater may be characterized by a very compact design. Such setup enables a possibility for prolonged off-grid situations.

The wind turbine may be an offshore wind turbine. In such embodiments, the foundation structure may include a gravity foundation or a monopile foundation or a tripod foundation or a jacket foundation or other off-shore facilities such as substations. In such embodiments, the gas may be also utilized to power offshore vessels such as vessels used for transporting crew and equipment to and from offshore wind turbines placed on tower support structure.

The wind turbine may be an onshore wind turbine. In such embodiments, a biogas reactor may be integrate is a separate space of the concrete foundation. Accumulated gas produced could be stored also in the foundation or a separate structure (in or above ground) or in the wind turbine tower itself.

Advantageously, according to the present disclosure, a very long off-grid time may be enabled and without need for refuelling if there is grid problems or under the offshore construction period.

According to embodiments of the present invention, the hollow portion is limited by a lateral inner surface of the foundation structure and two caps transversal to the lateral inner surface and distanced along an axis of the foundation structure. The two caps, respectively a bottom cap and a top, may be in particular provided on a foundation pile of an offshore wind turbine, the bottom cap and the top being distanced along a longitudinal axis of the foundation pile. Existing load optimized structure (thick walled steel structure) are utilized for multipurpose as energy storage as a tank.

According to the present invention, as "bioreactor" it is meant any container, which may receive and hold a biomass and generate, for example through a fermentation process, a biogas from the biomass. According to embodiments of the present invention, may include a bottom portion for receiving the biomass. The bottom portion may include a heater for promoting the transformation of the biomass into biogas. The heater may accelerate the fermentation process. The heater may be powered with electricity from the wind turbine when electric over-production occurs. It may also be powered from a power source that is generating electricity from the biogas already produced from the bioreactor. These features can be combined, so that the heater can be supplied with energy from either or both. According to other embodiments of the present invention, the heat supplied for the fermentation process may be supplied from excess heat generated from the cooling system of the turbine, e.g. generator excess heat or nacelle excess heat. Accordingly, excess heat on wind turbines which is now released to the environment (e.g. liquid-to-air heat exchanges placed at or within the turbine) may be saved for providing the energy to the bioreactor (e.g. working a liquid-to-liquid system).

According to embodiments of the present invention, the bioreactor includes at least a top portion for receiving the biogas produced from the biomass. After the transformation of the biomass is complete, the bioreactor, including the top and the bottom portions, may be completely filled with biogas. The top portion may include a hydrogen producing device for transforming the biogas into hydrogen using an electricity source as energy input. The hydrogen producing device may include a cathode and anode system using electricity preferably supplied from the wind turbine. This electricity by example supplied in a situation with excess power production and/or under negative electricity price periods.

The gas energy storage may be used to stabilize the grid when limited electricity from the turbine is needed. This enables turbines to be self-supported in a long period and evens the power. Gas for storage can be produced when there is no need for electricity. Thereby it may also help stabilizing the grid and storage of energy in a long time perspective.

According to embodiments of the present invention, the hollow portion may include one or more output ports for delivering the gas, e.g. to the gas electrical generator and/or to the gas heater and/or to the vessel, as above described.

It has to be noted that embodiments of the invention have been described with reference to different subject-matters. In particular, some embodiments have been described with reference to apparatus type claims whereas other embodiments have been described with reference to method type claims. However, a person skilled in the art will gather from the above and the following description that, unless otherwise notified, in addition to any combination of features belonging to one type of subject-matter also any combination between features relating to different subject-matters, in particular between features of the apparatus type claims and features of the method type claims is considered as to be disclosed with this application.

### Brief Description of the Drawings

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.
- Figure 1: shows a schematic section of an offshore wind turbine including a tower, a transition piece and a foundation monopile.
- Figure 2: shows a plurality alternative of foundation structures which may be used in embodiments of the present invention.
- Figure 3: shows a schematic functional representation of an embodiment of the present invention.
- Figure 4: shows a partial section view of the transition piece and the foundation monopile of figure 1.
- Figure 5: shows a partial section view of an alternative embodiment of a foundation monopile according to the present invention.
- Figure 6: shows another more detailed view of the foundation monopile of figure 5.
- Figure 7: shows a more detailed view of the transition piece and the foundation monopile of figure 4.
- Figure 8: shows an operative phase of an offshore wind turbine according to the present invention.
- Figure 9: shows another more detailed view of the foundation monopile of figure 5.
- Figure 10: shows a decommissioning phase of an offshore wind turbine including the foundation monopile of figures 5 and 9.

### Detailed Description

The illustrations in the drawings are schematically. It is noted that in different figures, similar or identical elements are provided with the same reference signs.

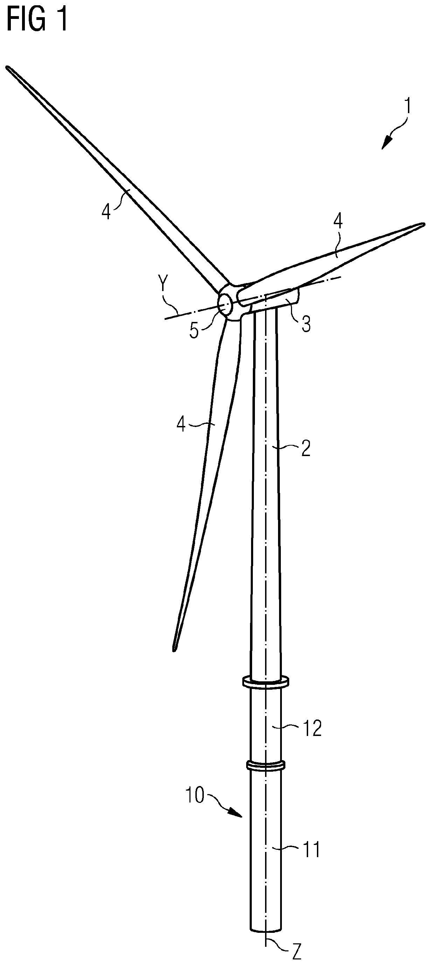

**Figure 1** shows an offshore wind turbine 1 according to the invention. The wind turbine 1 comprises a tower 2, which is mounted on a foundation structure 10. The foundation structure 10 is of the monopile type and includes a pile 11. The tower 2 and the pile 11 extend axially along a longitudinal axis Z of the offshore wind turbine 1. A nacelle 3 is attached to an upper end of the tower 2. The wind turbine 1 further comprises a wind turbine rotor 5 having three blades 4 (in the perspective of Figure 1 only two blades 4 are visible). The rotor 5 is attached to the nacelle 3 in order to be rotatable around a rotational axis Y. The foundation structure 10 comprises a transition piece 12 for connecting the tower 2 to the foundation pile 11. The transition piece 12 is attached to the bottom end of the tower 2, longitudinally opposite to the upper end of the tower 2. The wind turbine 1 comprises an electrical generator (not shown on figure 1), which is rotationally coupled with the wind rotor 5 for producing electrical energy to be provided to an electrical grid (not shown in the attached figures). According to embodiments of the present invention, the wind turbine 1 may be part of a wind turbine plant including a plurality of wind turbines 1.

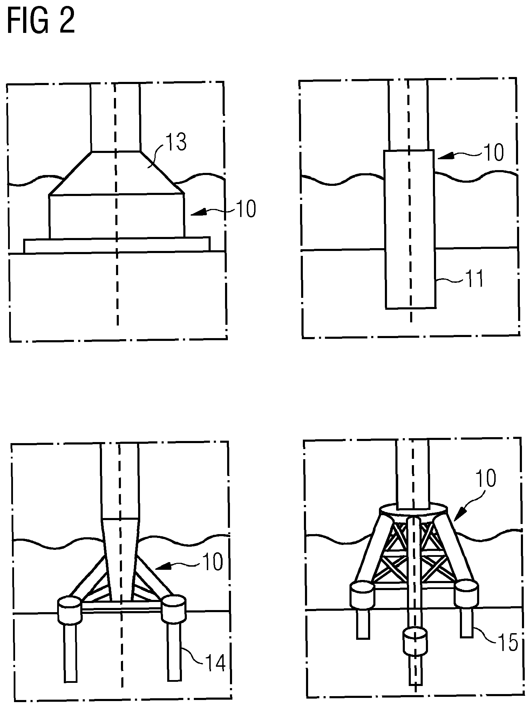

**Figure 2** shows four embodiments of the foundation structure 10. In a first embodiment, the foundation structure 10 includes a gravity foundation 13. In a second embodiment, the foundation structure 10 includes a single pile 11 (figure 1). In a third embodiment, the foundation structure 10 includes a tripod foundation 14. In a fourth embodiment, the foundation structure 10 includes or a jacket foundation 15. According to other embodiments, the offshore wind turbine 1 may include other types of foundation structure, for example offshore substations. According to other embodiments n, the present invention may be provided in the foundation structure of an onshore wind turbine.

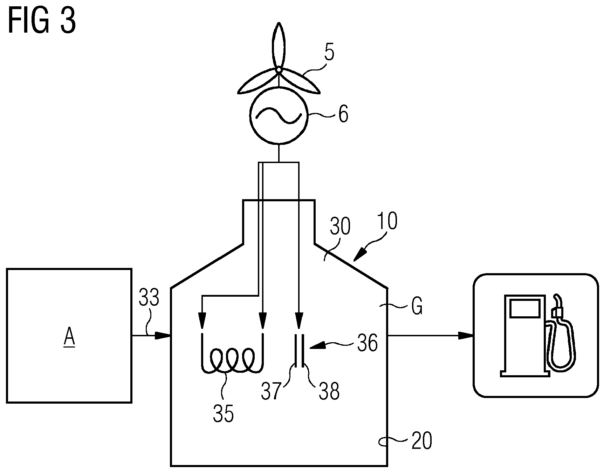

According to the functional simplified scheme of **Figure 3****,** of the foundation structure 10 comprises at least a hollow portion 20 and a bioreactor 30 inside the hollow portion 20. The bioreactor 30 comprises an input port 33 for receiving a biomass A, which may be a sea based organic material, for example seaweed. According to other embodiments, any type of biomass may be used inside the bioreactor 30. The bioreactor 30 generates a biogas G derived from the biomass A. The biogas G may include methane and any other gas derived from the transformation, for example by means of a fermentation process of the biomass A. The bioreactor 30 optionally includes a heater 35 for promoting the transformation of the biomass A into biogas G. The bioreactor 30 optionally further includes a hydrogen producing device 36 for transforming the biogas G into hydrogen using an electricity source as energy input. The hydrogen producing device 36 is of the electrolytic type including an anode 37 and a cathode 38. The heater 35 and the hydrogen producing device 36 are hydrogen producing device 36 are both connected to the electrical generator 6 of the wind turbine 1, in such a way to be supplied from the electricity produced by the wind turbine 1, in particular when electric over production occurs. According to other embodiments, the heater 35 and/or the hydrogen producing device 36 may be also be powered from a power source that is generating electricity from the biogas G already produced from the bioreactor. According to other embodiments of the present invention, the heat supplied for the fermentation process may be supplied from excess heat generated from the cooling system of the turbine, e.g. from the electric generator 6 or from the nacelle 3. According to embodiments of the present invention, cooling hoses from the nacelle 3 may be provided in the tower 2 and the transition piece 12 and guided to the bioreactor 30. In yet another embodiment, the heater 35 and/or the hydrogen producing device 36 may be powered by solar cells placed on the wind turbine 1 (outside the nacelle 3 or the tower 2). The bioreactor 30 stores the biogas G and/or the hydrogen produced by the hydrogen producing device 36. The bioreactor 30 includes an output port 34 for delivering the biogas G and/or the hydrogen produced by the hydrogen producing device 36. The biogas G and/or the hydrogen produced by the hydrogen producing device 36 may be used for a plurality of purposes, e.g. for fuelling a vehicle, such as a vessel and/or for operating a gas heater in the nacelle 3 or the tower 2 and/or for operating a gas electrical generator in the nacelle 3 or the tower 2.

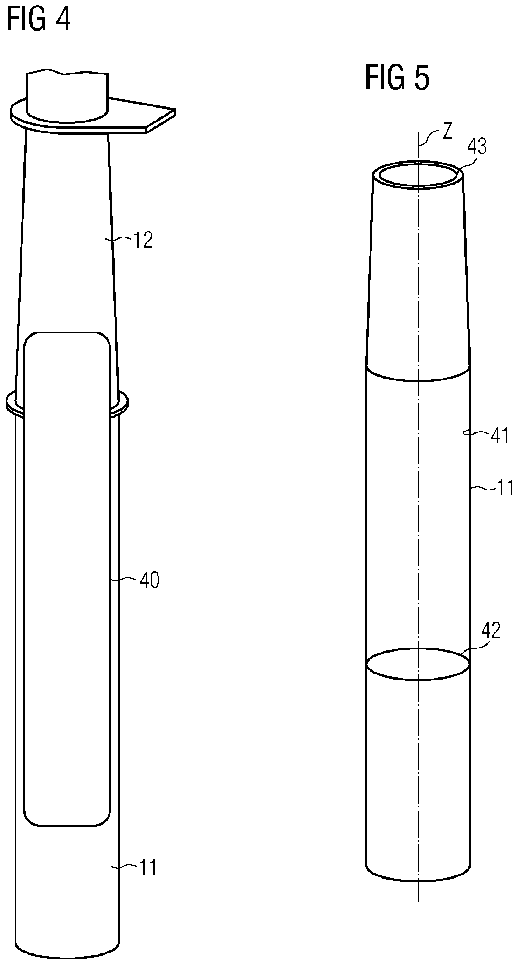

In a first embodiment (**Figure 4**), the bioreactor 30 is delimited by a substantially cylindrical container 40, housed inside the pile 11.

In a second embodiment (**Figure 5**), the bioreactor 30 is delimited by a lateral inner surface 41 of the pile 11, a bottom cap 42 and a top cap 43. The two caps 42, 43 are transversal to the lateral inner surface 41 and distanced along the axis Z of the pile 11. The two caps 42, 43 are fixed, for example by welding, to the lateral inner surface 41. The lateral inner surface 41 of the pile 11 and the two caps 42, 43 defines a substantially cylindrical enclosure for the bioreactor 30.

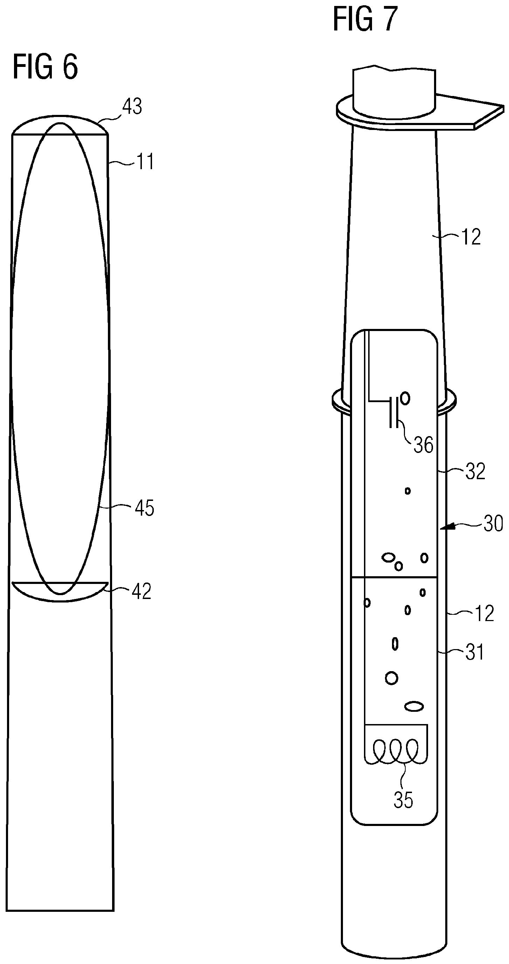

As shown in **Figure 6****,** the bioreactor 30 may include a corrosion protection on the lateral inner surface 41 of the pile 11, which is constituted by a plastic membrane 45, having the shape of a closed bag. The plastic membrane 45 may be inserted in the bioreactor 30 through an opening, for example a valve, of the bioreactor 30. The plastic membrane 45 may be inflated through the same opening to make it adhere to the the lateral inner surface 41 of the pile 11 (an intermediate stage of the inflation process is shown in figure 6). According to another embodiment, the corrosion protection may be provided by means of another material, for example a coating material painted on the lateral inner surface 41.

**Figure 7** shows more in detail the bioreactor 30. The bioreactor 30 includes a bottom portion 31 and a longitudinal opposite top portion 32. With reference to the embodiment of figure 5, the bottom portion 31 and the top portion 32 are respectively adjacent to the bottom cap 42 and the top cap 43. When the bioreactor 30 receives the biomass A from the input port 33, the biomass A following the gravity force fills first the bottom portion 31. The biomass A may fill completely the bioreactor 30, including the top portion 32. The filling of the bioreactor 30 may be performed, in offshore embodiments, by pumping see water including seaweed and/or alga through the input port 33. A filter may be provided to prevent impurities from entering the bioreactor 30. The bottom portion 31 may include the heater 35 for promoting the transformation of the biomass A into biogas G. The biogas starts to accumulate, following the gravity force, in the top portion 32. At the end of the transformation process the biogas G may fill completely the bioreactor 30, including the bottom portion 31. The top portion 32 may include the hydrogen producing device 36.

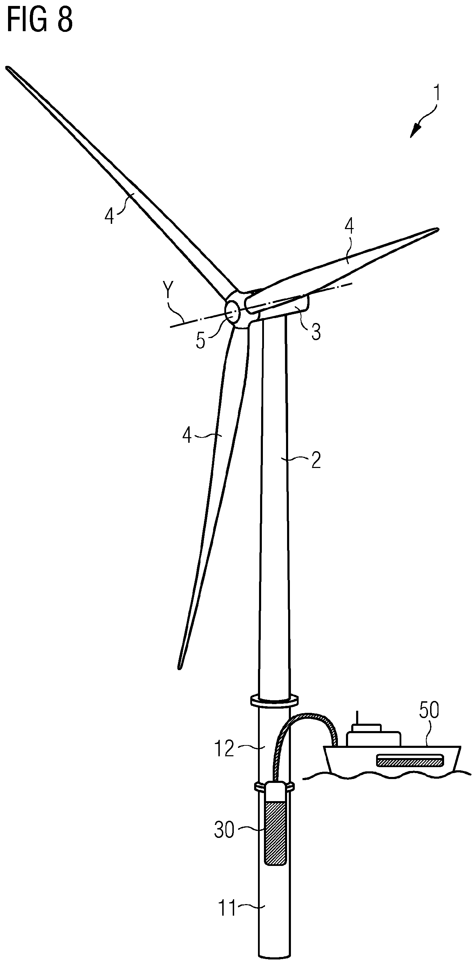

**Figure 8** shows a vessel being fueled with the biogas G from the bioreactor 30.

According to other embodiments of the present invention, the biogas produced and stored in the bioreactor 30 may also be used to power an electricity producing motor (combustion engine or hydrogen fuel cell). The biogas produced and stored in the bioreactor 30 may be methane or similar gases (e.g. alkane gases) or hydrogen. Thereby the wind turbine 1 may have its own electricity back-up support needed to power internals (cooling, lubrication pumps, etc.) for off-grid situations. In off grid extreme situations wind turbines are required to be able to yaw for six hours in order to survive wind gusts when a low pressure storm is passing. The electricity producing motor (combustion engine or hydrogen fuel cell) may then power the yaw system as an UPS. Battery solutions can thus be avoided as back-up support. In case of biogas production that exceeds the storage capacity, the biogas may be transported ashore or to separate storage facilities placed offshore and/or at or within the wind turbine tower 2.

According to other embodiments (not shown) of the present invention, the hollow portion 20 does not include a bioreactor, but includes an electrolytic hydrogen producing device for transforming a mass of water, for example sea water, into hydrogen using an electricity source as energy input. The hydrogen producing device may include a cathode and anode system using electricity preferably supplied from the wind turbine. The electrolytic hydrogen producing device may have a structure as disclosed in figures 4 and 5, i.e. delimited by a by a lateral inner surface 41 of the pile 11, a bottom cap 42 and a top cap 43. The hydrogen produced by the electrolytic hydrogen producing device may be used for any of the purposes above described with reference to the biogas.

According to other embodiments (not shown) of the present invention, the hollow portion 20 is used for storing a gas provided to the hollow portion from an external source, which is connected to the hollow portion 20 through a duct or a pipeline. The gas stored may be a biogas produced outside the hollow portion 20, or hydrogen or any other gas which may be used for any of the purposes above described with reference to the biogas.

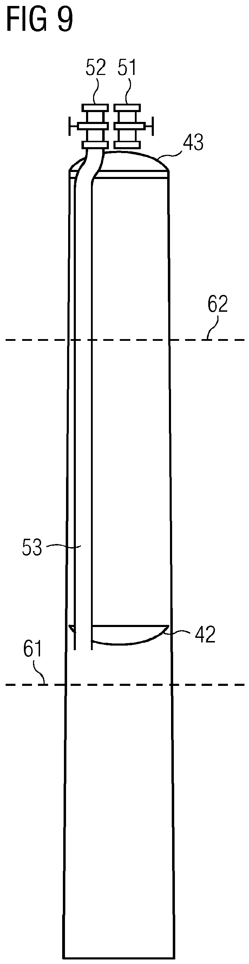

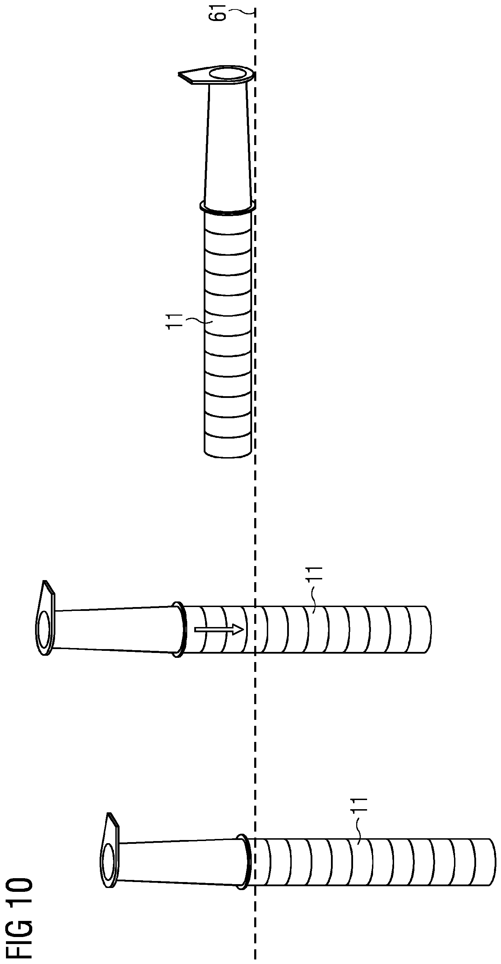

**Figures 9** and **10** show the possibility to efficiently decommission a foundation structure 10 including a bioreactor 30 according to the present invention. Figure 9 shows the pile 11 initially inserted in the seabed 61. The bottom cap 42 is above the seabed 61 while the see level 62 is comprised between he bottom cap 42 and the top cap 43. On the top cap 43 a top valve 51 and a bottom valve 52 are provided. The top valve 51 provides an input port to the bioreactor 30. The bottom valve 52 provides access through a duct 53 to the space between the hollow space between the bottom cap 42 and the seabed 61. The decommissioning can be performed by pumping a gas, e.g. air, in the hollow space between the bottom cap 42 and the seabed 61 through the bottom valve 52 and the duct 53. The compressed apply a force between the pile 11 (in particular the bottom cap 42) and the seabed 61 (left part of figure 10). The pile 11 moves up from the seabed 61 (central part of figure 10). At the end of such moving process, the pile 11 is fully up it will turn to horizontal (right part of figure 10) and can be removed by a vessel. For example, when the pile 11 is a float it can be transported away by only a tug boat.

## Claims

1. A wind turbine (1) including a foundation structure (10), a tower (2) and a hollow portion (20) for storing a gas (G),
wherein the hollow portion (20) is comprised in the foundation structure (10) or in the tower (2) of the wind turbine (1).

2. The wind turbine (1) according to claim 1, wherein the hollow portion (20) is limited by a lateral inner surface of the foundation structure (10) or of the tower (2) and two caps transversal to the lateral inner surface and distanced along an axis (Z) of the foundation structure or of the tower (2) .

3. The wind turbine (1) according to claim 1 or 2, further including a bioreactor (30) inside the hollow portion (20) for receiving a biomass (A) and generating a biogas (G) derived from the biomass (A).

4. The wind turbine (1) according to claim 3, wherein the bioreactor (30) includes at least a bottom portion (31) for receiving the biomass (A).

5. The wind turbine (1) according to claim 4, wherein the bottom portion (31) includes a heater (35) for promoting the transformation of the biomass (A) into biogas (G).

6. The wind turbine (1) according to claim 4 or 5, wherein the bioreactor (30) includes at least a top portion (32) for receiving the biogas (G).

7. The wind turbine (1) according to any of the previous claims, wherein the hollow portion (20) includes a hydrogen producing device (36).

8. The wind turbine (1) according to any of the previous claims, wherein the hollow portion (20) includes an output for (34) delivering the gas (G).

9. The wind turbine (1) according to any of the claims 2 to 8, wherein the foundation structure (10) includes a gravity foundation or a monopile foundation or a tripod foundation or a jacket foundation.

10. A method of generating and storing energy in a wind turbine (1) including the step of providing a hollow portion (20) in a foundation structure (10) or in a tower (2) of the wind turbine (1) for storing a gas (G).

11. A method of generating and storing energy in a wind turbine (1) including the steps of:
providing a bioreactor (30)
inserting a biomass (A) in the bioreactor (30),
using the bioreactor (30) for generating a biogas (G) from the biomass (A),
storing the biogas (G) in the hollow portion (20).

12. The method of claim 10 or 11, further including the step of:
using the gas (G) for fuelling a vehicle, such as a vessel.

13. The method of any of the claims 10 to 12, further including the step of:
using the gas (G) for operating a gas heater of the wind turbine (1).

14. The method of any of the claims 10 to 13, further including the step of:
using the gas (G) for operating a gas electrical generator.

15. The method of any of the claims 10 to 14, further including the step of:
using the gas (G) for operating a fuel cell.
